# EUROPEAN PATENT SPECIFICATION

(11) **EP 3 773 463 B1**
(45) Date of publication and mention of the grant of the patent: **27.10.2021**
(21) Application number: 19728080.3
(22) Date of filing: 06.06.2019
(51) Int. Cl.: A61K 8/67, A61K 8/60, A61K 8/92, A61K 8/34, A61K 8/91, A61K 8/81, A61Q 19/00

(54) **SKIN CARE KIT**
HAUTPFLEGEKIT
KIT DE SOINS CUTANÉS

(30) Priority: 03.07.2018 WO PCT/CN2018/094208; 14.08.2018 EP 18188831
(43) Date of publication of application: 17.02.2021
(73) Proprietor: Unilever Global IP Limited, Wirral, CH62 AZD (GB); Unilever IP Holdings B.V., 3013 AL Rotterdam (NL)
(72) Inventor: LIU, Jianfei, Shanghai, Changning District 200335 (CN)
(74) Representative: James, Helen Sarah
(86) International application number: PCT/EP2019/064786
(87) International publication number: WO 2020/007563

(56) References cited:
- EP-A1- 2 243 472
- WO-A1-2009/031788
- WO-A1-2015/032319
- FR-A1- 2 946 253
- US-A1- 2016 200 500

## Description

### Field of the invention

The present invention relates to a skin care kit. In particular, the present invention is related to a skin care kit comprising (a) a freeze-dried cosmetic composition comprising a cosmetic active, and (b) an aqueous cosmetic composition comprising an acrylic polymeric thickener, wherein the freeze-dried cosmetic composition comprises a polysaccharide.

### Background of the invention

A number of important cosmetic actives are known for being unstable due to external influence, for example light, oxidation and etc. The cosmetic actives may be altered or decomposed such that the product containing the actives may have a relatively short shelf life, or alternatively the functionality may be reduced during the lifetime of the product.

Therefore, there is big interest to develop new ways to include actives into cosmetic compositions which affords long-term stability over the entire shelf life and provides high efficiency in application. Freeze drying is a well-known and widely-used method particularly in pharmaceutics. With such a way, the cosmetic active substances may be included in a freeze-dried cosmetic composition (interchangeably with FDCC) in forms of powder, tablet and/or capsule, preventing the alteration or decomposition. Such FDCC may be coupled with an aqueous cosmetic composition (interchangeably with ACC). An end use skin care product may be generated in situ by mixing the FDCC with ACC just before use. In addition, such a FDCC may also include high dosage of actives to deliver a better efficiency and can also be shipped in large quantities to points of distribution for energy efficient manufacturing benefits.

However, we have recognized that when mixing the FDCC with some ACC, the generated end use skin care product may have some problem, for example, balling-up problem when it is spread onto skin. The present inventor(s) therefore developed a skin care kit comprising (a) a freeze-dried cosmetic composition comprising a cosmetic active, and (b) an aqueous cosmetic composition comprising an acrylic polymeric thickener, wherein the freeze-dried cosmetic composition comprises a polysaccharide. It was surprisingly found that the generated skin care product by the compositions of the kits have a good spreading sensory.

### Summary of the invention

In a first aspect, the present invention is directed to a skin care kit comprising (a) a freeze-dried cosmetic composition comprising a cosmetic active, and (b) an aqueous cosmetic composition comprising an acrylic polymeric thickener, wherein the freeze-dried cosmetic composition comprises a polysaccharide.

In a second aspect, the present invention is directed to a skin care product obtainable by mixing the freeze-dried cosmetic composition and the aqueous cosmetic composition of the present invention.

All other aspects of the present invention will more readily become apparent upon considering the detailed description and examples which follow.

### Detailed description of the invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

The cosmetic actives may comprise vitamin, sunscreen, resorcinol, or a mixture thereof. Preferably the cosmetic active comprises vitamin, vitamin derivatives, vitamin precursor, or a mixture thereof. More preferably, the cosmetic active comprises Vitamin B2, Vitamin B3 (niacinamide), Vitamin B6, ascorbic acid, derivatives thereof, and a mixture thereof. More preferably, the cosmetic active comprises ascorbic acid, ascorbic acid derivative or a mixture thereof. Even more preferably, the cosmetic active comprises ascorbic acid, ascorbyl tetraisopalmitate, magnesium ascorbyl phosphate, ascorbyl glycoside, or a mixture thereof. Still even more preferably, the cosmetic active comprises ascorbic acid and most preferably, the cosmetic active is ascorbic acid.

Preferably the cosmetic active is water-soluble. "Water-soluble" as used herein refers to ingredient that dissolves in water to give a solution with a concentration of at least 5 grams per liter at 25°C and atmospheric pressure.

Preferably, the cosmetic active is present in amount of 10 to 70% by weight of the FDCC, more preferably 25 to 60%, even more preferably from 30 to 55%, and still even more preferably 35 to 50% by weight of the FDCC.

In a preferred embodiment, the freeze-dried cosmetic composition comprises ascorbic acid in amount of 10 to 70% by weight of the FDCC, more preferably 25 to 60%, even more preferably from 30 to 55%, and still even more preferably 35 to 50% by weight of the FDCC.

The FDCC comprises a polysaccharide. Polysaccharide refers to polysaccharide having at least ten units of monosaccharide. The polysaccharide preferably comprises starch, gum, cellulosics, or a mixture thereof. Preferred starch is selected from tapioca starch, sodium hydroxypropyl starch phosphate, aluminium starch octenylsuccinate, or a mixture thereof. Suitable gum comprises Suitable gums include xanthan, sclerotium, pectin, karaya, arabic, agar, carrageenan, alginate or combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g. cellulose microfibrils, cellulose nanocrystals or microcrystalline cellulose)

More preferably the FDCC comprises a gum, even more preferably galactomannan. Preferably the galactomannan comprises guar gum, locust beam gum, cassia gum or a mixture thereof. More preferably, the galactomannan comprises guar gum and most preferably the galactomannan is guar gum.

Preferably the FDCC comprises polysaccharide in amount of 0.1 to 15%, more preferably 0.5 to 12%, even more preferably 0.8 to 8%, still even more preferably 1.2 to 5% by weight of the FDCC. Preferably the FDCC comprises galactomannan in amount of 0.1 to 15%, more preferably 0.5 to 12%, even more preferably 0.8 to 8%, still even more preferably 1.2 to 5% by weight of the FDCC. Preferably the FDCC comprises guar gum in amount of 0.1 to 15%, more preferably 0.5 to 12%, even more preferably 0.8 to 8%, still even more preferably 1.2 to 5% by weight of the FDCC.

To provide a better sensory of the end use skin care product, the weight ratio of the cosmetic active to the polysaccharide in the freeze-dried cosmetic composition is preferably 200:1 to 1:1, more preferably 80:1 to 2:1, and even more preferably 30:1 to 10:1.

To provide a better sensory of the end use skin care product, preferably, the FDCC comprises polyhydric alcohol. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. More preferably, the polyhydric alcohol comprises glycerin, propylene glycol, butylene glycol, or a mixture thereof. Preferably, the polyhydric alcohol has a molecular weight of 50 to 500, more preferably 60 to 300. Most preferably, the polyhydric alcohol comprises glycerin.

Preferably the polyhydric alcohol is present in amount of 10 to 50%, more preferably 18 to 38%, and even more preferably 22 to 32% by weight of the FDCC. More preferably the FDCC comprises glycerin in amount of 10 to 50%, more preferably 18 to 38%, and most preferably 22 to 32% by weight of the FDCC.

Preferably the FDCC comprises oligosaccharide. Oligosaccharide as used herein refers to saccharide containing 1 to 9 monosaccharide units. More preferably the FDCC comprises a disaccharide. Even more preferably, the FDCC comprises a trehalose. Preferably, the amount of the disaccharide is 3 to 50%, more preferably 10 to 45%, even more preferably 16 to 35% and still even more preferably 22 to 30% by weight of the FDCC.

To provide better sensory of the end use skin care product, preferably the weight ratio of the cosmetic active to the polyhydric alcohol is from 1:3 to 8:1, more preferably from 1:1 to 3:1 and even more preferably from 1.2:1 to 1.8:1. Preferably, the weight ratio of the polyhydric alcohol to the polysaccharide is from 1:1 to 30:1, more preferably 2:1 to 20:1, and even more preferably 5:1 to 15:1. The weight ratio of the cosmetic active to the disaccharide is preferably from 3:1 to 8:1, more preferably 0.6:1 to 4:1, and even more preferably from 1.2:1 to 2:1. The weight ratio of the polyhydric alcohol to the disaccharide is preferably from 1:8 to 8:1, more preferably 1:4 to 4:1, and even more preferably from 1:1.5 to 1.5:1. The weight ratio of the galactomannan to the disaccharide is preferably from 1:20 to 3:1, more preferably from 1:15 to 1:1, and even more preferably from 1:10 to 1:3.

The FDCC may comprise water, preferably in amount of less than 10% by weight of the FDCC, more preferably 0.1 to 8% and most preferably 3 to 6% by weight of the FDCC. Preferably the FDCC is substantially free of surfactant. "substantially free of surfactant" means that a surfactant is completely absent or, if any, present in the FDCC in an amount of less 0.1% by weight of the FDCC.

The FDCC may comprise preservatives although it is also within the scope of the invention that the FDCC is preservative free. Particularly preferred preservatives are iodopropynyl butyl carbamate, phenoxyethanol, 1,2-octanediol, hydroxyacetophenone, ethylhexylglycerine, hexylene glycol, methyl paraben, propyl paraben, imidazolidinyl urea, sodium dehydroacetate and benzyl alcohol. It is more preferred that the preservative is combination of 1,2-octanediol and phenoxyethanol, or combination of iodopropynyl butyl carbamate and phenoxyethanol. Preservatives are preferably employed in amounts ranging from 0.01% to 5%, more preferably 0.1 to 3% even more preferably 0.2 to 2% based on the total weight of the freeze-dried cosmetic composition.

Fragrances, fixatives and exfoliants may optionally be included in the FDCC the present invention. Each of these substances may range from 0.05 to 5%, preferably between 0.1 and 3% by weight of the FDCC.

The FDCC is obtainable by freeze drying (lyophilizing) a freeze-dried cosmetic FDCC precursor. All features except the amount of the ingredient by weight of the FDCC (including the weight ratio between ingredients except with water) described for the freeze-dried cosmetic composition are considered also apply to the FDCC precursor.

Preferably, the precursor is not an emulsion. In a preferred embodiment, the precursor has a viscosity from 750 to 55,000 cps, and more preferably, from 2,000 to 40,000 cps, and most preferably, from 5,000 to 30,000 cps, including all ranges subsumed therein, where the viscosity of the precursor may be measured with a Brookfield (DV-1+) Viscometer, temperature 25°C and set at 20 RPM, RV6 for 30 seconds.

The FDCC precursor may be freeze dried (lyophilization) by any art recognized technique. Such a technique includes the steps of first freezing precursor in a freezer at about -17 to -20°C. Optionally the precursor can be frozen in liquid nitrogen (-196°C) or in a mixture of acetone and dry ice (-80°C) by shell freezing in the round bottom flask. The frozen precursors can then be transferred to a freeze dryer. The precursor is freeze-dried preferably at temperature of from -20 to -90°C, more preferably -25 to -50°C. Preferably, the freeze drying is conducted at vacuum of preferably 0.07 to 1.8 mbar, and more preferably from 0.08 to 1.3 mbar to drive off water.

The shape or form of the resulting freeze-dried cosmetic composition is dependent on the shape and size of the container or form carrying or holding the precursor during the freeze-drying process. Preferably, the freeze-dried cosmetic composition is in form of powder, flake, capsule, or tablet. More preferably, the FDCC is in form of capsule or tablet. Most preferably, the FDCC is in form of tablet.

The aqueous cosmetic composition (interchangeable with ACC) refers to composition comprising at least 20%, preferably at least 30% of water by weight of the aqueous composition. Preferably, the aqueous cosmetic composition comprises 40 to 90%, more preferably 44 to 70% of water by weight of the aqueous composition. It should be noted that the FDCC is physically separate from the ACC.

The acrylic polymeric thickeners as used herein means polymeric thickener comprising, as polymerized monomer, acrylic acid, its homologues, and/or derivatives thereof. Preferably, the acrylic polymeric thickener comprises, as polymerized monomer, acrylic acid, methacrylic acid, esters of acrylic acid, esters of methacrylic acid, acrylonitrile, acrylamide, cyanoacrylates, combination thereof, or derivatives thereof. More preferably, the acrylic polymeric thickener comprises polyacrylates, polyacrylamides, polymethacrylate, acrylate copolymer, methacrylate copolymer, acrylamide copolymer, acryloyldimethyltaurate copolymer, or a mixture thereof. Even more preferably, the acrylic polymeric thickener comprises polyacrylate, polyacrylamide, acryloyldimethyltaurate copolymer, acrylate copolymer or a mixture thereof. Still even more preferably, the acrylic polymeric thickener comprises Carbomer, Acryloyl Dimethyltaurate/Vinyl Pyrrolidone Copolymer, or a mixture thereof.

Preferably, the acrylic polymeric thickener is present in the ACC in amount of 0.01 to 8%, more preferably 0.08 to 5%, even more preferably 0.3 to 3%, and still even more preferably 0.5 to 2% by weight of the aqueous cosmetic composition.

The aqueous cosmetic composition preferably comprises water-soluble ingredient. Preferably, the water-soluble ingredient is stable in water. Preferably, the ACC comprises polyol. Polyols may be selected from group of propylyene glycol, dipropylene glycol, polypropylene glycol, polyethylene glycol, sorbitol, hydroxypropyl sorbitol, hexylene glycol, 1,3-butylene glycol, isoprene glycol, ethoxylated glycerol, propoxylated glycerol or a mixture thereof. More preferably the ACC comprises glycerin, propylene glycol, dipropylene glycol, butylene glycol, or a mixture thereof. Most preferably the ACC comprises polyhydric glycerol known also as glycerin. The amount of the polyol in the ACC is preferably 5 to 50%, more preferably 12 to 35%, and even more preferably 18 to 28% by weight of the aqueous cosmetic composition.

Preferably, the weight ratio of the polyol to the acrylic polymeric thickener is 2:1 to 200:1, more preferably 4:1 to 120:1 and even more preferably 15:1 to 60:1.

A small amount of emulsifying surfactant may be present in the ACC. Surfactants may be anionic, nonionic, cationic, amphoteric, or mixtures thereof but preferably the ACC comprises a nonionic surfactant. The non-ionic surfactant is preferably selected from the group comprising fatty alcohol ethoxylates, fatty acid ethoxylates, alkyl phenol ethoxylates, polyoxyethylene sorbitan alkyl esters, polyalkyleneglycol ether of fatty acid glyceride or partial glyceride or a mixture thereof. More preferred non-ionic surfactants comprise fatty alcohol ethoxylate, polyalkyleneglycol ether of fatty acid glyceride or partial glyceride, or a mixture thereof. Levels may range, for example, from 0.1 to 5%, more preferably from 0.1 to 3%, optimally from 0.1 to 2% by weight of the ACC. Advantageously the amount of surfactant present should not be sufficient for lather formation. In these instances, less than 2% by weight, preferably less than 1%, and optimally less than 0.5% by weight surfactant is present.

Preferably the ACC is substantially free of gum thickener, preferably substantially free of polysaccharide thickener. "Substantially free of" means that it is completely absent or, if any, present in the ACC in an amount of less 0.1%, preferably less than 0.01% by weight of the ACC. Preferably the ACC is substantially free of magnesium aluminum silicate and more preferably substantially free of silicate.

It is preferable that the freeze-dried cosmetic composition is provided inside a first cosmetics container. Preferably, the aqueous cosmetic composition is provided inside a separate packaging unit. The separate packaging unit may be part of the first cosmetic container, or a second cosmetic container. More preferably, the aqueous cosmetic composition is provided inside a second cosmetic container.

The first cosmetic container may be selected from bottle, jar, box, and/or blister pack depending on the form of the FDCC. It is preferably, the FDCC is in form of tablet and the cosmetic container is a blister pack. The second cosmetic container is preferably a jar or bottle.

Preferably the kit comprises an instruction for use of the kit. The consumers may prepare a final skin care product just before use by mixing the freeze-dried cosmetic composition with an aqueous cosmetic composition according to the instruction. Preferably, the skin care product is produced in 1 second to 5 minutes, more preferably in 10 seconds to 2 minutes. The instruction preferably includes guidance on the way of mixing, and more preferably, includes guidance on the amounts of the FDCC and the ACC to be added, and even more preferably include guidance on the amounts of the FDCC and the ACC to be mixed and the desired mode of agitation of the mixture.

The present invention also provides a skin care product for end use obtainable by mixing the freeze-dried cosmetic composition of the present invention with the aqueous composition. Preferably, the weight ratio of the FDCC to the ACC is preferably 1:100 to 5:1 more preferably 1:40 to 2:1, even more preferably 1:15 to 1:1 when making the skin care product for end use.

It is preferable that the final skin care product comprises the cosmetic active in amount of at least 3% by weight of the product, more preferably 5 to 25%, even more preferably 7 to 15% by weight of the product.

The skin care product refers to a product suitable for topical application to human skin, including leave-on and wash-off products. Preferably the skin care product is a leave-on product. The term "leave-on" as used herein means a product that is applied to or rubbed on the skin, and left thereon. The term "skin" as used herein includes the skin on the face (except eye lids and lips), neck, chest, abdomen, back, arms, under arms, hands, and legs. Preferably "skin" includes the skin on the face (except eye lids and lips) and under arms. More preferably skin means skin on the face other than lips and eyelids.

The following examples are provided to facilitate an understanding of the invention. The examples are not intended to limit the scope of the claims.

### Examples

### Example 1

This example demonstrates the preparation of a freeze-dried cosmetic tablets.

**Table 1**

| Ingredient | wt % |
|---|---|
| Ascorbic acid | 41.74 |
| Trehalose | 26.08 |
| Glycerin | 26.08 |
| Guar Gum | 2.09 |
| Water | 4.01 |

The freeze-dried cosmetic composition in Table 1 was prepared by freezing a freeze-dried cosmetic composition precursor in liquid nitrogen, and then being subjected to a freeze dryer for freeze drying. The freeze-dried cosmetic composition was made in tablet form with a weight of 50 mg.

### Example 2

This example demonstrates the preparation of skin care products for end use.

**Table 2**

| Ingredient | Aqueous cosmetic compositions (wt%, active level) | | | | | | | |
|---|---|---|---|---|---|---|---|---|
| | A | B | C | D | 1 | 2 | 3 | E |
| Water | 99.00 | 99.00 | 99.00 | 99.00 | 99.00 | 99.00 | 75.00 | 75.00 |
| Glycerin | - | - | - | - | - | - | 10.00 | 10.00 |
| Butylene Glycol | - | - | - | - | - | - | 10.00 | 10.00 |
| Ethanol | - | - | - | - | - | - | 3.00 | 3.00 |
| Carpylic/Capric Triglyceride | - | - | - | - | - | - | 1.00 | 1.00 |
| Xanthan Gum ^{a} | 1.00 | - | - | - | - | - | - | 1.00 |
| Caesalpinia Spinosa Gum ^{b} | - | 1.00 | - | - | - | - | - | - |
| Sclerotium gum ^{c} | - | - | 1.00 | - | - | - | - | - |
| Magnesium aluminum silicate ^{d} | - | - | - | 1.00 | - | - | - | - |
| Ammonium Acryloyldimethyltaur ate/VP copolymer ^{e} | - | - | - | - | 1.00 | - | 1.00 | - |
| Carbomer ^{f} | - | - | - | - | - | 1.00 | - | - |

| | | | | | | | | |
|---|---|---|---|---|---|---|---|---|
| a: Keltrol® CG-SFT xanthan gum, supplied by CP Kelco b: SOLAGUM™ TARA, supplied by SEPPIC c: Amigel®, supplied by Alban Muller International d: VEEGUM® Magnesium Aluminum Silicate, supplied by Vanderbilt Minerals, LLC e: Aristoflex® AVC, supplied by Clariant f: Carbopol® Ultrez 20 polymer, supplied by Lubrizol | | | | | | | | |

The aqueous cosmetic compositions in Table 2 were prepared by following standard way. The freeze-dried cosmetic tablets were mixing with the aqueous compositions of Table 2 by human hands. The times of dissolving the tablet into different aqueous cosmetic compositions were recorded. Then, the sensory of the end-use skin care products was assessed when it is applied to skin by human hands. It was found that the skin care products generated by mixing freeze-dried cosmetic tablet with aqueous cosmetic composition A, B, C, D, and E respectively formed crumbs or pills on the skin, indicating that they had balling-up problem. In contrast, the skin care products generated by mixing freeze-dried cosmetic tablet with aqueous cosmetic composition 1 to 3 were easily spread onto the skin. The results are shown in Table 3. In addition, the tablet dissolves quicker in the aqueous cosmetic composition of the present invention (1 to 3) than others (A to E).

**Table 3**

| Sample | Sensory of the end use product |
|---|---|
| A | Balling-up |
| B | Balling-up |
| C | Balling-up |
| D | Balling-up |
| 1 | Easy to spread |
| 2 | Easy to spread |
| 3 | Easy to spread |
| E | Balling-up |

## Claims

1. A skin care kit comprising:
(a) a freeze-dried cosmetic composition comprising a cosmetic active; and
(b) an aqueous cosmetic composition comprising an acrylic polymeric thickener,
wherein the freeze-dried cosmetic composition comprises a polysaccharide.

2. The kit according to claim 1 wherein the cosmetic active comprises ascorbic acid, ascorbic acid derivatives, or a mixture thereof, preferably the cosmetic active comprises ascorbic acid.

3. The kit according to claim 1 or 2 wherein the cosmetic active is present in amount of 10 to 70% by weight of the freeze-dried cosmetic composition, preferably 25 to 60%, more preferably 35 to 52% by weight of the freeze-dried cosmetic composition.

4. The kit according to any one of the preceding claims wherein the freeze-dried cosmetic composition comprises guar gum.

5. The kit according to claim 4 wherein the polysaccharide is present in amount of 0.1 to 15%, preferably 0.5 to 12%, more preferably 1.2 to 5% by weight of the freeze-dried cosmetic composition.

6. The kit according to claims 4 or 5 wherein the weight ratio of the cosmetic active to the polysaccharide in the freeze-dried cosmetic composition is 200:1 to 1:1, preferably 80:1 to 2:1, and more preferably 30:1 to 10:1.

7. The kit according to any one of the preceding claims wherein the freeze-dried cosmetic composition comprises polyhydric alcohol, disaccharide or a mixture thereof, preferably the freeze-dried cosmetic composition comprises polyhydric alcohol and disaccharide.

8. The kit according to any one of the preceding claims wherein the freeze-dried cosmetic composition is substantially free of surfactant.

9. The kit according to any one of the preceding claims wherein the acrylic polymeric thickener comprises polyacrylate, polyacrylamide, acrylate copolymer, or a mixture thereof.

10. The kit according to any one of the preceding claims wherein the acrylic polymeric thickener is present in amount of 0.01 to 8%, preferably 0.3 to 3% by weight of the aqueous cosmetic composition.

11. The kit according to any one of the preceding claims wherein the aqueous cosmetic composition comprises polyol.

12. The kit according to any one of the preceding claims wherein the amount of the polyol is 5 to 50%, preferably 12 to 35% by weight of the aqueous cosmetic composition.

13. The kit according to claim 11 or 12 wherein the weight ratio of the polyol to the acrylic polymeric thickener is 2:1 to 200:1, preferably 15:1 to 60:1.

14. The kit according to any one of the preceding claims wherein the kit comprises an instruction for use of the kit.

15. A skin care product obtainable by mixing the free-dried cosmetic composition of any one of the preceding claims and the aqueous cosmetic composition of any one of the preceding claims.

## Patentansprüche

1. Hautpflegekit, umfassend:
(a) eine gefriergetrocknete kosmetische Zusammensetzung, umfassend einen kosmetischen Wirkstoff; und
(b) eine wässrige kosmetische Zusammensetzung, umfassend ein polymeres Acrylverdickungsmittel,
wobei die gefriergetrocknete kosmetische Zusammensetzung ein Polysaccharid umfasst.

2. Kit nach Anspruch 1, wobei der kosmetische Wirkstoff Ascorbinsäure, Ascorbinsäurederivate oder eine Mischung davon umfasst, wobei der kosmetische Wirkstoff bevorzugt Ascorbinsäure umfasst.

3. Kit nach Anspruch 1 oder 2, wobei der kosmetische Wirkstoff in einer Menge von 10 bis 70 Gewichts-% der gefriergetrockneten kosmetischen Zusammensetzung, bevorzugt 25 bis 60 Gewichts-%, bevorzugter 35 bis 52 Gewichts-%, der gefriergetrockneten kosmetischen Zusammensetzung vorliegt.

4. Kit nach irgendeinem der vorhergehenden Ansprüche, wobei die gefriergetrocknete kosmetische Zusammensetzung Guargummi umfasst.

5. Kit nach Anspruch 4, wobei das Polysaccharid in einer Menge von 0,1 bis 15 Gewichts-%, bevorzugt 0,5 bis 12 Gewichts-%, bevorzugter 1,2 bis 5 Gewichts-%, der gefriergetrockneten kosmetischen Zusammensetzung vorliegt.

6. Kit nach den Ansprüchen 4 oder 5, wobei das Gewichtsverhältnis des kosmetischen Wirkstoffs zum Polysaccharid in der gefriergetrockneten kosmetischen Zusammensetzung 200:1 bis 1:1, bevorzugt 80:1 bis 2:1 und bevorzugter 30:1 bis 10:1 beträgt.

7. Kit nach irgendeinem der vorhergehenden Ansprüche, wobei die gefriergetrocknete kosmetische Zusammensetzung mehrwertigen Alkohol, Disaccharid oder eine Mischung davon umfasst, wobei die gefriergetrocknete kosmetische Zusammensetzung bevorzugt mehrwertigen Alkohol und Disaccharid umfasst.

8. Kit nach irgendeinem der vorhergehenden Ansprüche, wobei die gefriergetrocknete kosmetische Zusammensetzung im Wesentlichen frei von Tensid ist.

9. Kit nach irgendeinem der vorhergehenden Ansprüche, wobei das polymere Acrylverdickungsmittel Polyacrylat, Polyacrylamid, Acrylatcopolymer oder eine Mischung davon umfasst.

10. Kit nach irgendeinem der vorhergehenden Ansprüche, wobei das polymere Acrylverdickungsmittel in einer Menge von 0,01 bis 8 Gewichts-%, bevorzugt 0,3 bis 3 Gewichts-%, der wässrigen kosmetischen Zusammensetzung vorliegt.

11. Kit nach irgendeinem der vorhergehenden Ansprüche, wobei die wässrige kosmetische Zusammensetzung Polyol umfasst.

12. Kit nach irgendeinem der vorhergehenden Ansprüche, wobei die Menge des Polyols 5 bis 50 Gewichts-%, bevorzugt 12 bis 35 Gewichts-%, der wässrigen kosmetischen Zusammensetzung beträgt.

13. Kit nach Anspruch 11 oder 12, wobei das Gewichtsverhältnis des Polyols zum polymeren Acrylverdickungsmittel 2:1 bis 200:1, bevorzugt 15:1 bis 60: 1, beträgt.

14. Kit nach irgendeinem der vorhergehenden Ansprüche, wobei das Kit eine Anweisung zur Verwendung des Kits umfasst.

15. Hautpflegeprodukt, erhältlich durch Mischen der gefriergetrockneten kosmetischen Zusammensetzung nach irgendeinem der vorhergehenden Ansprüche und der wässrigen kosmetischen Zusammensetzung nach irgendeinem der vorhergehenden Ansprüche.

## Revendications

1. Kit pour le soin de la peau comprenant :
(a) une composition cosmétique lyophilisée comprenant un actif cosmétique ; et
(b) une composition cosmétique aqueuse comprenant un épaississant polymère acrylique, dans lequel la composition cosmétique lyophilisée comprend un polysaccharide.

2. Kit selon la revendication 1, dans lequel l'actif cosmétique comprend de l'acide ascorbique, des dérivés d'acide ascorbique, ou un mélange de ceux-ci, l'actif cosmétique comprend de préférence de l'acide ascorbique.

3. Kit selon la revendication 1 ou 2, dans lequel l'actif cosmétique est présent dans une quantité de 10 à 70 % en masse de la composition cosmétique lyophilisée, de préférence de 25 à 60 %, encore mieux de 35 à 52 % en masse de la composition cosmétique lyophilisée.

4. Kit selon l'une quelconque des revendications précédentes, dans lequel la composition cosmétique lyophilisée comprend de la gomme guar.

5. Kit selon la revendication 4, dans lequel le polysaccharide est présent dans une quantité de 0,1 à 15 %, de préférence de 0,5 à 12 %, encore mieux de 1,2 à 5 % en masse de la composition cosmétique lyophilisée.

6. Kit selon la revendication 4 ou 5, dans lequel le rapport massique de l'actif cosmétique au polysaccharide dans la composition cosmétique lyophilisée est de 200:1 à 1:1, de préférence 80:1 à 2:1, et encore mieux 30:1 à 10:1.

7. Kit selon l'une quelconque des revendications précédentes, dans lequel la composition cosmétique lyophilisée comprend un alcool polyvalent, un disaccharide ou un mélange de ceux-ci, la composition cosmétique lyophilisée comprend de préférence un alcool polyvalent et un disaccharide.

8. Kit selon l'une quelconque des revendications précédentes, dans lequel la composition cosmétique lyophilisée est substantiellement exempte de tensioactif.

9. Kit selon l'une quelconque des revendications précédentes, dans lequel l'épaississant polymère acrylique comprend un polyacrylate, polyacrylamide, copolymère d'acrylate, ou un mélange de ceux-ci.

10. Kit selon l'une quelconque des revendications précédentes, dans lequel l'épaississant polymère acrylique est présent dans une quantité de 0,01 à 8 %, de préférence de 0,3 à 3 % en masse de la composition cosmétique aqueuse.

11. Kit selon l'une quelconque des revendications précédentes, dans lequel la composition cosmétique aqueuse comprend un polyol.

12. Kit selon l'une quelconque des revendications précédentes, dans lequel la quantité du polyol est de 5 à 50 %, de préférence de 12 à 35 % en masse de la composition cosmétique aqueuse.

13. Kit selon la revendication 11 ou 12, dans lequel le rapport massique du polyol à l'épaississant polymère acrylique est de 2:1 à 200:1, de préférence de 15:1 à 60:1.

14. Kit selon l'une quelconque des revendications précédentes, dans lequel le kit comprend une instruction pour l'utilisation du kit.

15. Produit pour le soin de la peau pouvant être obtenu en mélangeant la composition cosmétique lyophilisée selon l'une quelconque des revendications précédentes et la composition cosmétique aqueuse selon l'une quelconque des revendications précédentes.
